# FASCICULE DE BREVET EUROPEEN

(11) **EP 3 193 724 B1**
(45) Date de publication et mention de la délivrance du brevet: **25.11.2020**
(21) Numéro de dépôt: 15749802.3
(22) Date de dépôt: 11.08.2015
(51) Int. Cl.: A61B 6/14, A61B 6/00

(54) **PROCEDE ET SYSTEME DE PRISE D'IMAGES RADIOLOGIQUES MEDICALES AVEC COMMANDE D'ARRET DE LA SOURCE DE RAYONNEMENT X**
VERFAHREN UND ANORDNUNG ZUR AUFNAHME VON MEDIZINISCHEN RÖNTGENBILDERN MIT STEUERUNG DES STOPPVORGANGS DER RÖNTGENQUELLE
METHOD AND SYSTEM FOR TAKING MEDICAL RADIOLOGY IMAGES WITH CONTROL OF THE STOPPING OF THE X-RAY SOURCE

(30) Priorité: 02.09.2014 FR 1458200
(43) Date de publication de la demande: 26.07.2017
(73) Titulaire: Teledyne e2v Semiconductors SAS, 38120 Saint-Egrève (FR)
(72) Inventeur: JULIEN, Florian, Mid Levels (HK); LUSSEREAU, Laurent, F-38112 Meaudre (FR); PELLET, Pascal, F-38000 Grenoble (FR); DELZOPPO, Yves, F-38120 Saint Egrève (FR)
(74) Mandataire: Marks & Clerk France
(86) Numéro de dépôt international: PCT/EP2015/068492
(87) Numéro de publication internationale: WO 2016/034379

(56) Documents cités:
- EP-A1- 1 857 049
- EP-A2- 2 180 343
- US-A1- 2013 077 744
- US-A1- 2013 223 592
- US-B1- 6 404 854

## Description

### DOMAINE TECHNIQUE

L'invention concerne un procédé et un système de prise d'images radiologiques médicales, et notamment dentaires, permettant de minimiser le temps d'exposition d'un patient aux rayonnements X, tout en s'assurant d'une prise d'image de bonne qualité.

### ETAT DE LA TECHNIQUE

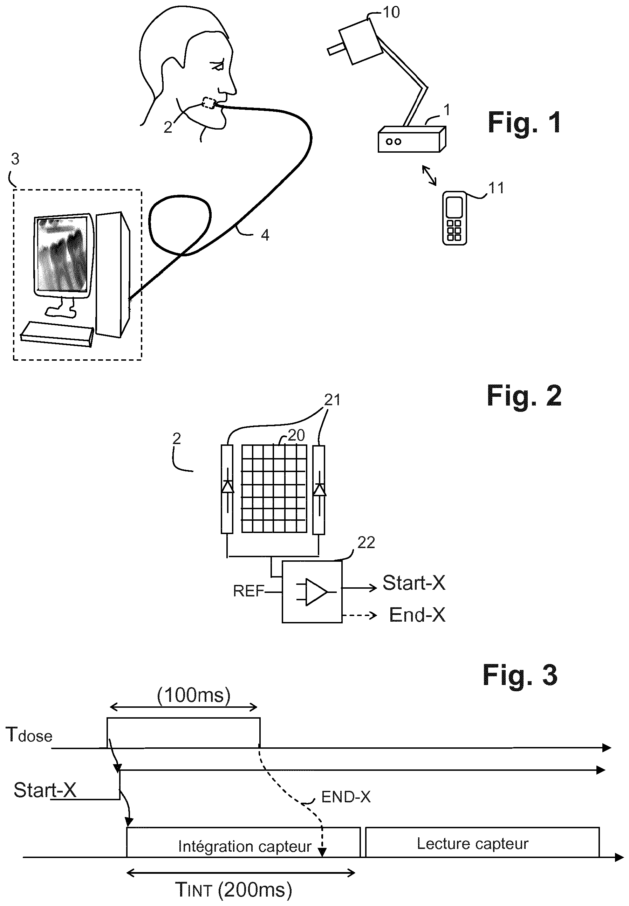

Un système de radiologie médicale comprend comme schématiquement illustré sur la figure 1, une source 1 de rayonnement X, un capteur d'image 2 sensible au rayonnement X et un dispositif 3 de traitement et visualisation des images fournies par le capteur.

Dans l'exemple illustré, il s'agit d'un système de radiologie dentaire. Le capteur d'image est placé à l'intérieur de la bouche du patient, derrière l'objet, la dent, à radiographier. On parle de capteur intra-oral. La source de rayons X est munie d'une tête orientable 10 pour émettre un rayonnement vers la joue du patient, en direction du capteur. La dose de rayons X est préalablement paramétrée par le praticien au moyen d'un boitier de contrôle tel qu'une télécommande 11. Le capteur transmet les données d'image collectées au dispositif 3 de traitement et visualisation, qui est généralement dans ce cas un ordinateur (de bureau, portable...) du praticien. Dans l'invention on s'intéresse plus particulièrement aux systèmes dans lesquels le capteur et l'ordinateur communiquent par une liaison filaire 4, de type USB.

On sait que les capteurs d'image CMOS ont fait une large percée remplaçant dans nombre de domaines les capteurs CCD. C'est le cas également dans le domaine qui nous occupe. Dans les systèmes de radiologie dentaire les plus récents, on propose ainsi comme capteur intra-oral, un capteur CMOS qui se connecte à l'ordinateur par une liaison filaire 4 répondant au protocole USB (*Universal Serial Bus).* Le brevet EP1146559 en décrit un exemple. L'utilisation d'une interface USB est très avantageuse. Le capteur devient un simple "périphérique USB" au même titre qu'une imprimante, un scanner.... Il peut être facilement connecté/déconnecté de n'importe quel ordinateur du cabinet dentaire ayant le logiciel pilote adéquat. La consommation en énergie d'un capteur intra-oral CMOS étant faible, il est aussi alimenté via la liaison USB par l'ordinateur. On évite ainsi les modules d'alimentation spécifiques (piles).

### PROBLEME TECHNIQUE

Une problématique récurrente des systèmes de radiologie dentaire, et plus généralement de radiologie médicale, est celle de la durée du rayonnement X. Cette durée doit être suffisante pour pouvoir obtenir une image de bonne qualité, c'est-à-dire ni sous exposée, ni sur exposée. En même temps, la durée d'exposition du patient, du praticien aussi, au rayonnement X doit être la plus faible possible.

Pour répondre à cette problématique les sources de rayonnement X ont été améliorées pour optimiser les caractéristiques du rayonnement et réduire le dosage nécessaire. Des améliorations ont aussi été apportées pour en faciliter le paramétrage par le praticien en fonction de la zone d'intérêt (quelle dent) et la masse du patient. Pour chaque prise d'image, une dose correspondante est définie (tension (kV), courant (mA), durée d'exposition (ms)) en fonction des paramètres positionnés par le praticien en fonction du patient (âge) et de la dent concernés. Il reste que ce paramétrage n'est pas parfait, selon que le praticien a plus ou moins d'expérience, mais aussi parce le type de dent et l'âge du patient ne sont pas des informations tout à fait suffisantes pour paramétrer parfaitement la dose à utiliser. Ainsi le risque d'obtenir une image sous exposée ou sur exposée est-il important.

Une autre difficulté est liée à la manière par laquelle les différents éléments du système inter-opèrent. En effet le capteur et la source sont des appareils autonomes l'un de l'autre ; il n'y a pas de canal de communication directe entre eux. Aussi, une fois paramétrée, lorsque le dentiste déclenche ensuite la source (via la télécommande 11 - Figure 1) celle-ci émet la dose définie de rayons X pendant le temps d'exposition déterminé. Mais le capteur n'a pas cette information de début d'exposition (ni de fin) de manière directe.

Or pour avoir une image de meilleure qualité possible, avec le moins de bruit possible, la phase d'intégration du capteur ne doit démarrer que lorsque le capteur reçoit effectivement le rayonnement. Avant, le capteur ne perçoit que du bruit, en particulier le bruit d'obscurité des éléments photosensibles des pixels.

C'est ainsi que dans ces systèmes de prise d'image radiographique, les capteurs comprennent habituellement des moyens de détection d'un rayonnement X. Dès qu'il détecte un rayonnement, par des moyens propres, intégrés, il déclenche la phase d'intégration.

Différentes techniques de détection sont utilisées en pratique. Parmi elles, on peut citer celle schématiquement illustrée sur la figure 2, qui consiste à prévoir en périphérie de la matrice 20 de pixels du capteur 2, un ensemble 21 de photodiodes de détection couplées à des moyens 22 de lecture et comparaison à un seuil REF prédéterminé. Dans ce cas, on prévoit généralement deux colonnes de photodiodes en série, une de chaque côté de la matrice de pixels photosensibles. Le courant fourni par ces photodiodes est comparé au seuil REF prédéterminé. En l'absence de radiation, le signal correspond essentiellement à du bruit, principalement le courant d'obscurité. En présence d'un rayonnement X le niveau de signal augmente sensiblement. Dès que le seuil REF est dépassé, un signal X-Start indiquant le début de rayonnement est activé. L'unité de commande du capteur (non représenté) qui fournit les différents signaux de séquencement pour les phases d'intégration et de lecture des pixels, reçoit ce signal Start-X et lance la phase d'intégration du capteur.

Cette phase d'intégration a une durée préprogrammée TINT, à une valeur choisie, par exemple 200 ms égale ou légèrement supérieure à la durée d'exposition maximale possible avec la source X utilisée. Typiquement, l'activation du signal Start-X signalant le début d'exposition au rayonnement déclenche un compteur programmé pour décompter la durée TINT. Au terme du décomptage, la période d'intégration se termine et la phase de lecture du capteur et transfert des données image est déclenchée.

Dans certains capteurs, on prévoit aussi de détecter la fin du rayonnement X, en se basant sur le fait que lorsque le rayonnement va s'arrêter, le niveau de signal ne va plus évoluer. Par exemple, le même ensemble de photodiodes de la figure 2 peut être utilisé, la détection consistant à regarder si le niveau de signal continue à évoluer ou pas. De cette manière on peut émettre un signal End-X indiquant la fin de l'émission du rayonnement X. Ce signal permet de terminer la phase d'intégration pour enclencher la lecture, sans attendre la fin de la durée TINT. D'autres techniques de détection de fin d'émission du rayonnement peuvent être utilisées. Par exemple le brevet US 6,404,854B décrit un procédé de lecture non destructive de pixels de la matrice, pour détecter que le niveau de signal de certains pixels de la matrice n'évolue plus, indiquant que la source a arrêté d'émettre.

Ces détections effectuées dans le capteur permettent ainsi de récupérer l'information de temps d'exposition du rayonnement émis par la source et de caler le début d'intégration, voire la fin d'intégration du capteur sur le temps d'exposition réel de la source de rayonnement, limitant la part de bruit dans les données du capteur. La figure 3 représente un chronogramme avec un temps d'émission de rayons X (Tdose, par exemple 100 ms), et un temps d'intégration (TINT, par exemple 200 ms) du capteur avant une lecture du signal intégré. Le temps d'intégration est déclenché par le début d'émission et se termine après la fin de l'émission.

Mais on a vu que la dose paramétrée dans la source peut ne pas être "optimale" en terme de qualité d'image. Notamment, le temps d'exposition Tdose paramétré dans la source de rayons X peut être en réalité légèrement trop long, ce qui conduit alors à une saturation des pixels. L'image sera sur-exposée.

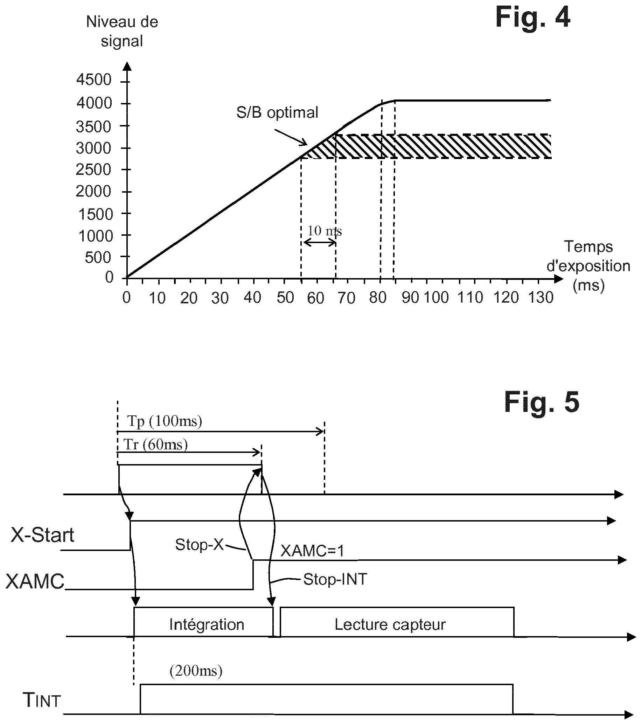

Il n'y a en effet pas d'état intermédiaire entre la zone de fonctionnement linéaire et la zone de saturation des pixels, comme illustré sur la figure 4. La zone de fonctionnement du capteur correspondant à un rapport signal sur bruit optimal est indiquée en hachuré sur cette figure : de manière bien connue, elle correspond à un niveau de signal des pixels qui correspondant à environ ¾ de la dynamique du capteur, et entre le moment où l'on rentre dans cette zone, et celui où on sort de cette zone, il ne s'écoule que peu de temps, dans l'exemple 10 ms. Dans l'exemple on obtient un bon rapport signal sur bruit après 55 ms d'exposition à la dose de rayonnement, et à partir de 80 ms, on commence à entrer dans la zone de saturation. Ainsi, avec un temps d'exposition au rayonnement X légèrement trop long, on passe en un court laps de temps, d'une image de bonne qualité, à une image saturée en niveaux de gris, avec absence de contrastes dans la ou des zones d'intérêt. Or si l'image est saturée, elle ne sera pas exploitable. Il faudra donc refaire la prise d'image, ce qui suppose de la paramétrer à nouveau, pour réduire la dose et aussi de soumettre le patient à une nouvelle dose de rayonnement. En outre, le praticien perd du temps à refaire ces réglages et ces nouveaux clichés.

On voit bien que qualité d'image et nécessité de soumettre le patient à une dose minimale de rayonnement sont des problématiques très liées. Ainsi il ne suffit pas de pouvoir paramétrer la source ; et il ne suffit pas que le capteur soit à même de détecter le début et la fin du temps d'exposition de la dose émise par la source externe. Il faudrait encore permettre au capteur d'arrêter cette source externe de manière dynamique, pendant le processus de prise d'image, et plus spécialement pendant la phase d'intégration, lorsque le niveau de signal obtenu sur au moins une partie des pixels du capteur correspond à un niveau optimal.

Il est connu de prévoir dans des capteurs, et notamment dans des capteurs CMOS, un contrôle du temps d'exposition pour éviter la saturation des pixels, par lecture répétée et non destructive d'au moins certains pixels de la matrice pendant la période d'intégration. Ces pixels choisis sont lus de manière non destructive, et le niveau de signal obtenu est comparé à un seuil déterminé correspondant, défini en fonction des caractéristiques du capteur. Lorsque ce seuil est atteint ou dépassé pour un certain nombre de ces pixels "témoins", on décide que l'exposition au rayonnement est suffisante et assure une bonne qualité d'image. Un signal correspondant est activé.

Dire que la lecture de ces pixels est non destructive veut dire que ces pixels ne sont pas réinitialisés après la lecture : ils continuent à intégrer des charges. Ainsi l'information de ces pixels n'est pas perdue. L'unité de contrôle du capteur qui assure le séquencement des différentes phases en délivrant les signaux d'adressage et d'activation des circuits de lecture et conversion analogique du capteur est alors configurée pour permettre une telle lecture non destructive répétée pendant la phase d'intégration.

Cette lecture non destructive peut être effectuée pour tout type de structure de pixel CMOS, mais son implémentation est facilitée dans le cas d'une structure à trois transistors (pixels 3T), dans laquelle c'est le niveau de signal obtenu directement de l'élément photosensible qui est lu (et non celui d'un puits de stockage associé). Si on prend ainsi l'exemple d'un capteur CMOS à pixels à 3 transistors (pixel 3T), avec lecture par double échantillonnage corrélé (CDS) et conversion analogique numérique, la lecture CDS des pixels comprend de manière classique les phases suivantes : chaque pixel est initialisé au début de la phase d'intégration, et un niveau d'initialisation du pixel est mémorisé. A la fin de la phase d'intégration, la photodiode du pixel contient une quantité de charges représentative de l'éclairement reçu par la photodiode dans cette phase. On lit le pixel pour obtenir un niveau de signal représentatif de cet éclairement. La lecture CDS réalise une soustraction entre le niveau d'initialisation et ce niveau de signal, pour obtenir une mesure représentative de l'éclairement mais débarrassé d'une composante de bruit corrélé. Selon les capteurs, cette soustraction peut être réalisée de manière analogique ou de manière numérique. Le pixel est ensuite ré-initialisé pour la période d'intégration suivante.

Si on ne ré-initialise pas le pixel, on effectue une lecture nondestructive : le pixel va continuer d'accumuler des charges avec celles qu'il a déjà accumulées. On peut donc vérifier en continu le niveau de signal pendant toute la phase d'intégration, sans perdre d'information.

La demande US2005/0253944 met en œuvre une telle lecture non destructive dans un capteur CMOS, pour arrêter l'exposition à la lumière en activant un obturateur ou en coupant la source de lumière intégrée (Leds), dans un contexte où la source de lumière est intégrée ou liée au capteur, et notamment dans une capsule endoscopique à capteur CMOS.

Dans l'invention, on souhaite pouvoir arrêter la source de rayons X sur la base d'une telle information fournie par le capteur, mais dans le contexte décrit et illustré en figure 1, d'une source de rayonnement externe et autonome du capteur, dans lequel l'on ne dispose pas d'une liaison ou communication directe entre le capteur et la source de rayonnement et où le capteur est connecté comme périphérique USB d'un ordinateur hôte.

Le problème technique qui se pose dans ce contexte particulier, est que l'on ne sait pas transmettre cette information de manière suffisamment rapide ni de manière suffisamment sûre vers la source.

En effet, le protocole de communication USB ne prévoit pas un mode de communication rapide d'un évènement particulier, qui permette à un périphérique USB d'avertir, c'est-à-dire d'interrompre immédiatement, l'ordinateur hôte.

Le protocole USB prévoit bien un mode dit de transfert "d'interruption", mais il ne s'agit pas d'interruption au sens commun du terme, c'est-à-dire pour que l'ordinateur arrête tout processus en cours pour traiter en priorité une interruption.

Ce mode de transfert dit d'interruption du protocole USB permet seulement de transmettre des données particulières, peu nombreuses, typiquement des événements, comme par exemple l'événement de début d'exposition Start-X, qui pourra être utilisé par l'ordinateur dans le traitement des données d'image qu'il recevra ensuite.

Dans ce mode de transfert d'interruption du protocole USB, l'ordinateur vient en réalité interroger régulièrement le périphérique, à une fréquence dite de "polling" définie pour le périphérique concerné, pour savoir si le périphérique a un "évènement" à transférer selon ce mode. En réponse, le périphérique renvoie une trame de données, avec l'événement ou les évènements qu'il a à transmettre. En pratique, dans la trame, l'événement est codé sous forme d'un numéro d'événement (ce codage des évènements fait partie des descripteurs du périphérique connus de l'ordinateur).

Avec un tel mode de communication, on ne sait pas en pratique relayer suffisamment rapidement l'information vers la source pour la stopper. Dans le meilleur des cas Il faut compter au mieux vingt millisecondes à compter du moment où l'événement est disponible (détecté) dans le capteur, et relayé à la source par l'ordinateur. Comme on peut le voir sur la figure 4, vingt millisecondes, c'est déjà trop : on sera déjà passé dans la zone de saturation du ou des pixels. Et dans le pire cas, l'événement peut même ne pas être traité, spécialement en cas de panne informatique fortuite de l'ordinateur.

### RESUME DE L'INVENTION

L'invention est définie par les revendications 1 et 7. L'idée à la base de l'invention est d'instaurer une boucle de réaction entre le capteur et la source de rayons X pour stopper l'émission des rayons X dès qu'une dose suffisante a été reçue par le capteur, en plaçant un équipement espion sur la liaison USB qui va détecter l'événement correspondant émis par le capteur dans le flux de données transmis sur la liaison, et transmettre un signal électrique directement à la source, pour stopper le rayonnement. L'équipement espion effectue la détection de manière transparente pour le capteur et l'ordinateur hôte.

Plus précisément, l'invention concerne un procédé et un système de prise d'image en radiologie médicale, le système comprenant une source de rayonnement X externe et un capteur d'image CMOS relié par une liaison USB en tant que périphérique USB d'un dispositif de traitement et visualisation des données images du capteur. Le procédé comprend une lecture et un décodage continu des données transmises sur la liaison USB, d'une manière transparente pour le dispositif et pour le capteur, pour détecter dans le flux des données transmises, une donnée particulière (XAMC=1) représentant un signal de détection par le capteur qu'une dose de rayonnement suffisante a été reçue et, sur détection de cette donnée particulière, l'émission d'un signal électrique vers la source de rayonnement pour stopper l'émission du rayonnement. Le procédé est notamment applicable à une prise d'image radiologique dentaire par un capteur CMOS intra-oral.

De préférence, au moins un autre signal électrique est émis vers la source, pour commander un voyant d'affichage.

La donnée particulière à détecter et décoder est une donnée transmise par le capteur sur la liaison USB selon un mode de transfert par interruption.

Le système de prise d'image selon l'invention comprend une source de rayonnement X externe, un capteur à matrice de pixels CMOS disposé en vis-à-vis de la source, et un dispositif de traitement numérique des images fournies par le capteur, dans lequel le capteur est connecté comme périphérique USB dudit dispositif de traitement, par une liaison série USB. Il est caractérisé en ce que :
- le capteur est configuré pour détecter qu'une dose suffisante de rayons X a été reçue et transmettre une donnée particulière correspondante sur la liaison USB ;
- un équipement espion est connecté sur la liaison USB, entre le capteur et le dispositif, ledit équipement ayant en outre une connexion électrique avec la source, pour transmettre au moins un signal électrique (Stop-X) à la source pour commander l'arrêt du rayonnement X, ledit équipement étant apte à détecter ladite donnée particulière dans le flux des données transmises sur la liaison USB, et activer le dit signal électrique (Stop-X) commandant l'arrêt de la source.

Selon un autre aspect de l'invention, le procédé comprend de préférence :
- la définition de N régions d'intérêt sur la matrice de pixels du capteur, N entier au moins égal à deux, où les régions d'intérêt définies sont des sous-matrices de pixels d'égales dimensions, avec toutes le même nombre de lignes et le même nombre de colonnes,
- une lecture répétée non destructive des pixels de chacune des régions d'intérêt pendant la durée de la phase d'intégration du capteur, avec pour chacune des régions d'intérêt, le calcul d'une valeur moyenne du niveau de signal lu sur les pixels d'une ou plusieurs régions d'intérêt et la comparaison à une valeur seuil prédéterminée, et
- un signal de détection indiquant qu'une dose de rayonnement suffisante a été reçue est activé par le capteur et une information correspondante est transmise sur la liaison USB par le capteur dès que pour une ou plusieurs des N régions d'intérêt, la valeur moyenne calculée dépasse la valeur seuil.

Le procédé est avantageusement utilisé pour la prise d'image radiologique dentaire au moyen d'un capteur d'image CMOS intra-oral.

D'autres caractéristiques et avantages de l'invention apparaîtront à la lecture de la description détaillée qui suit et qui est faite en référence aux dessins annexés dans lesquels :
- la figure 1, déjà décrite, représente un système de radiologie dentaire ;
- la figure 2 représente schématiquement un capteur intra-oral intégrant des moyens de détection d'un début d'exposition aux rayons X;
- la figure 3 déjà décrite est un chronogramme des signaux montrant d'une part le temps d'exposition paramétré dans la source, et l'activation des phases d'intégration et lecture dans le capteur de la figure 2 ;
- la figure 4 déjà décrite illustre la sensibilité du capteur en fonction de la durée d'exposition au rayonnement X ;
- la figure 5 est un chronogramme des signaux utilisés dans l'invention pour contrôler la durée d'exposition du capteur en vue de garantir une image de qualité optimale et un temps d'exposition minimal ;
- la figure 6 illustre un système de radiologie dentaire selon l'invention, comprenant un équipement espion placé sur la liaison USB entre le capteur et l'ordinateur ;
- la figure 7 est un schéma bloc d'un équipement espion selon l'invention ;
- la figure 8 est un schéma illustrant un capteur avec des régions d'intérêt définies sur la matrice de pixels pour détecter une durée d'exposition suffisante au rayonnement X ; et
- la figure 9 est un organigramme montrant un exemple d'une boucle de détection de durée d'exposition suffisante au rayonnement X par lecture non destructive dans le capteur du ou des pixels d'une ou de régions d'intérêt définies sur un capteur tel qu'illustré à la figure 8.

### DESCRIPTION DETAILLEE DE L'INVENTION

Les figures 5 à 9 qui illustrent l'invention utilisent pour les éléments en commun, les mêmes références que dans les figures déjà décrites 1 à 4.

Le procédé de prise d'image radiologique utilise un capteur 2 d'image intra-oral, CMOS, qui transmet un signal d'information XAMC au dispositif 3 indiquant qu'une dose suffisante de rayonnement a été reçue, où la notion de suffisance s'entend par rapport à une qualité d'image optimale.

Selon l'invention, et comme illustré sur le chronogramme de la figure 5, le procédé de prise d'image utilise ce signal XAMC d'une manière qui va être décrite en détail ci-après, pour arrêter aussitôt la source de rayonnement X, au moyen d'un signal Stop-X, sans passer par le dispositif hôte, dont la prise en charge est trop lente compte-tenu des durées en cause.

Le temps d'intégration du capteur s'arrête ultérieurement lorsque la source cesse d'émettre des rayons X ; le capteur détecte lui-même l'absence d'illumination lorsque la source n'émet plus (détection par des photodiodes telles que 21 par exemple) et il déclenche l'arrêt de l'intégration. La lecture des signaux intégrés peut alors être effectuée. La phase d'intégration s'arrêtera donc (Stop-INT) en général avant la durée préprogrammée TINT. On a vu en effet que cette durée TINT est programmée en fonction des caractéristiques de la source de rayonnement, à une valeur légèrement supérieure au temps d'exposition maximale de cette source. Dans l'exemple illustré (Figure 5), on arrête ainsi et la source et la phase d'intégration au bout d'un temps Tr, ici environ 60ms après le début de l'exposition au rayonnement X, alors que la dose paramétrée dans la source prévoyait un temps d'exposition Tp de 100ms. Egalement, la phase de lecture peut démarrer tout de suite après, sans attendre la fin du décomptage de la durée programmée de temps d'intégration TINT (ici 200ms).

Ainsi, dans un processus de prise d'image selon l'invention, la phase d'intégration du capteur peut avantageusement débuter avec un signal Start-X généré en interne par le capteur, et qui correspond à la détection du début de la réception du rayonnement X, et se termine avec celui des deux évènements suivants qui arrive en premier : l'interruption de la source suite au passage au niveau actif (un dans l'exemple) du signal XAMC, indiquant qu'une dose suffisante de rayonnement a été reçue ou bien la fin du comptage (décomptage) de la durée maximale programmée TINT depuis le début de la phase d'intégration.

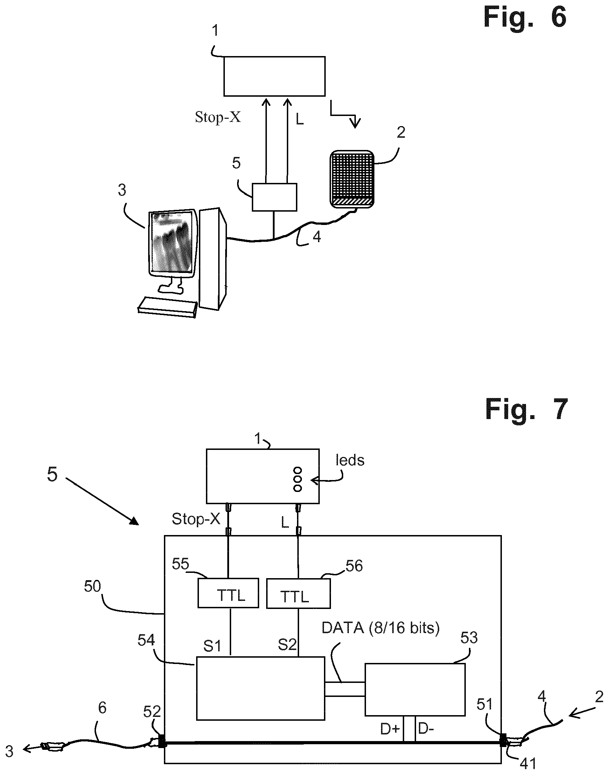

Ce procédé de prise d'image permettant d'optimiser la qualité d'image avec une exposition minimale du patient au rayonnement, est obtenu en utilisant un équipement espion 5 sur la liaison USB 4 entre le capteur et l'ordinateur, comme illustré sur la figure 6.

Il s'agit pour cet équipement de détecter, de manière transparente pour le capteur et le dispositif hôte 2, c'est-à-dire sans perturber la communication entre ces deux appareils, la transmission par le capteur de l'information qui correspond à l'activation du signal XAMC.

Rappelons que lorsque le signal XAMC est activé (XAMC=1 dans l'exemple), un évènement correspondant, que l'on note pour simplifier, évènement XAMC, est transmis par le capteur, plus précisément par un circuit d'interface USB implémenté dans le capteur, vers l'ordinateur hôte 3, sur la liaison USB 4. L'événement XAMC est transmis sur le bus USB via un « EndPoint » dédié, dans le protocole USB, à la gestion des évènements. Il possède un code spécifique permettant de le distinguer d'autres événements.

L'équipement espion 5 a ainsi pour fonction de détecter la transmission d'un évènement XAMC dans le flux de données circulant sur la liaison USB 4, sans perturber cette liaison, c'est-à-dire sans interrompre ou perturber la communication entre l'ordinateur et le capteur. La détection consiste à lire toutes les données transitant sur la paire différentielle de la liaison USB et à les décoder pour y détecter les données figurant sur le « Endpoint » dédié à la gestion des évènements dont le numéro d'événement XAMC correspondant.

Quand l'équipement espion fait cette détection, il génère un signal électrique Stop-X à la source, et plus précisément à l'unité de contrôle de la source, ce qui a pour effet d'arrêter le rayonnement. On peut aussi prévoir que l'équipement espion transmettra au moins un autre signal électrique L, par exemple pour allumer un voyant géré par l'unité de contrôle de la source. Il est habituel en effet que les sources de rayons X soient munies d'un certain nombre de voyants (des Leds) pour des raisons de sécurité et d'information (pour le praticien ou un assistant), pour indiquer si la source est en train d'émettre ou pas ... Il est donc intéressant de pouvoir allumer (ou éteindre) un voyant correspondant de la source, quand on en commande l'arrêt.

En pratique, les signaux délivrés en sortie par l'équipement espion seront par exemple des signaux logiques TTL, transmis par câble coaxial reliant l'équipement et la source. Le connecteur (BNC, DIN1.0/2.3, ou autre) utilisé pour connecter ce câble sur la source, dépend en pratique des interfaces de connexion proposées sur la source utilisée.

Un tel équipement espion 5 ne pose pas de difficultés pratiques de réalisation. L'homme du métier peut utiliser des circuits électroniques du commerce, comme schématiquement présenté sur la figure 7.

Il est formé d'un boitier 50 qui comprend un connecteur, par exemple un connecteur de type BNC, pour la connexion à la source de rayonnement 1, pour la transmission du signal Stop-X, et aussi du signal L le cas échéant ; un connecteur USB femelle 51, pour recevoir le connecteur mâle USB 41 de l'interface USB 4 du capteur ; un connecteur femelle USB 52, pour la connexion USB 6 à l'ordinateur 3. Entre les deux connecteurs femelles 51 et 52, on a les 4 fils habituels de la liaison USB : masse, tension logique, et les deux fils de données D- et D+ (notés aussi DM et DP dans la littérature technique).

A l'intérieur du boitier, une carte électronique comprend :
- un circuit du commerce 53 qui est un émetteur/récepteur (encore appelé dans la littérature technique *"transceiver"* USB, haute vitesse, qui comprend de manière classique un port d'interface série sur lequel on relie les deux fils de la paire différentielle D- et D+ de la liaison USB. Il peut ainsi lire et décoder les trames de signaux USB D- et D+ qui circulent sur la liaison USB sans interférer, et il effectue une conversion série parallèle pour fournir en sortie des données numériques, sur un bus de sortie parallèle DATA (de 8 ou 16 bits).
- un circuit logique programmable 54, par exemple un circuit FPGA *(field-programmable gate array),* programmé pour :
- détecter dans ces données transmises sur le bus DATA par le circuit 53 les trames de type transfert d'interruption et dans ces trames, l'événement XAMC, et
- activer en sortie un ou des signaux binaires correspondants. Dans l'exemple, le circuit 54 va activer à 1 un premier signal S1 et un deuxième signal S2 quand il détecte l'évènement XAMC dans la suite de données transmises sur le bus parallèle DATA.
- un étage de sortie par signal logique. Ce sont les circuits notés 55 et 56 sur la figure. Ils assurent une fonction d'adaptation électrique de chacun des signaux logiques S1 et S2, en fonction de l'interface électrique avec la source ainsi qu'une fonction d'isolation électrique du circuit logique programmable 54. Typiquement, ce sont des étages de sortie de signaux TTL.

Le boitier 50 est alimenté par une alimentation externe branché sur le secteur.

Le procédé et le système de prise d'image radiographique qui viennent d'être décrit dans le cadre d'une application plus spécialement dentaire, peut être élargi au contexte médical plus généralement, dès lors que le capteur d'image utilisé est relié par une liaison USB à un dispositif hôte, qui reçoit et traite les données image du capteur.

Ce procédé et ce système reposent sur l'utilisation d'un signal d'information XAMC généré dans le capteur et sur la détection de la transmission d'un évènement correspondant sur la liaison USB.

Le signal XAMC est généré dans le capteur par toute technique connue de l'homme de l'art, par comparaison du niveau de signal d'un ou de pixels de la matrice à un seuil fixé en fonction de la dynamique et de la sensibilité du capteur, pour correspondre à un niveau de signal optimal, non saturé.

De préférence, il est généré par lecture non destructive de ces pixels. De cette façon l'information de ces pixels peut être utilisée ensuite dans les données image. Il n'y a pas de perte d'information pour l'image.

Ce principe de lecture non destructive aux fins de test du niveau de signal des pixels est connu de l'homme de l'art. On pourra par exemple se reporter aux publications déjà citées précédemment US2005/0253944 et US 6,404,854 B, pour plus de détails.

En pratique, cette lecture non destructive est conduite par l'unité de contrôle du capteur, qui assure le séquencement nécessaire des signaux de commande pour adresser les différents pixels à tester, les connecter aux circuits de lecture et de conversion analogique numérique et assurer la transmission des données numériques obtenus. De manière conventionnelle, les circuits de lecture et de conversion analogique numérique permettent une lecture dite CDS.

Comme déjà expliqué, dans la phase d'intégration, l'unité de contrôle pilote ces circuits pour venir lire en boucle au moins certains pixels, sans réinitialisation après lecture. Pour chaque lecture effectuée, la donnée lue est comparée à un seuil ; cette comparaison est par exemple réalisée de manière numérique dans un circuit logique programmable de type FPGA habituellement prévu sur le capteur pour traiter les données numériques obtenues des pixels. Le seuil est positionné pour un capteur donné pour correspondre de préférence aux ¾ de la dynamique du capteur. On peut prévoir que le seuil soit programmé à une valeur par défaut, mais paramétrable par le praticien, via l'ordinateur et la liaison USB. Quand pour au moins un pixel, et de préférence pour plusieurs pixels, le niveau de signal obtenu dépasse le seuil, le circuit logique programmable active en sortie un signal d'information binaire XAMC correspondant, par exemple, en le passant au niveau "1", si on reprend la convention retenue sur le chronogramme de la figure 5.

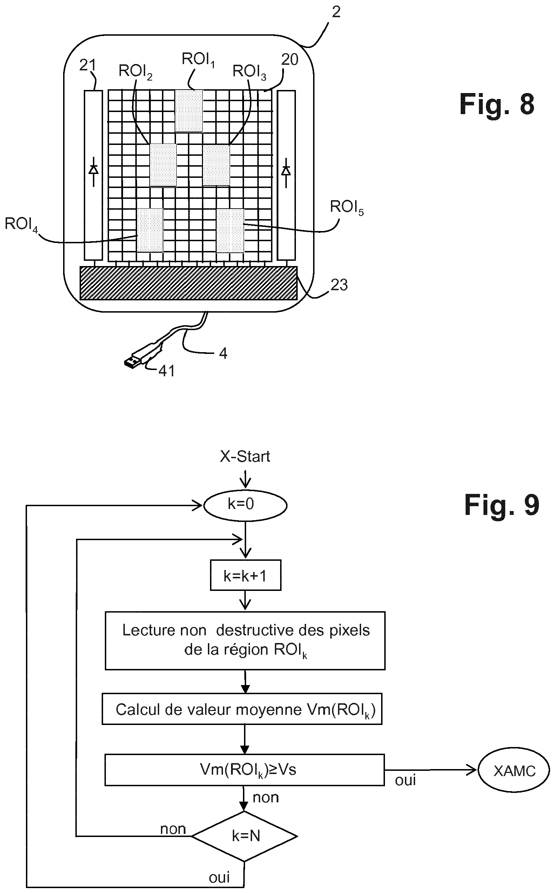

Les figures 8 et 9 montrent un exemple de réalisation préférée de la détection effectuée par le capteur, de la réception d'une dose suffisante de rayonnement. Il faut comprendre par dose suffisante, une dose par laquelle le niveau de signal atteint par un certain nombre de pixels correspond à un niveau optimal, non saturé, comme précédemment expliqué en relation avec la figure 4.

La détection à effectuer doit en effet de préférence être rapide, réactive, puisque comme on l'a vu, on passe très rapidement de la zone optimale de rapport signal sur bruit, à une zone de saturation de signal. Elle doit aussi être fiable. Il ne suffit pas de l'information d'un seul pixel. Il en faut plusieurs. Mais on ne peut pas lire tous les pixels de la matrice car cela prendrait trop de temps en regard des temps d'exposition moyens utilisés.

En effet, si on considère par exemple un capteur intra oral à 1.6 millions de pixels, une séquence de lecture complète à 5MHz nécessite par exemple 381ms, ce qui est du même ordre de grandeur que les temps d'exposition à une dose de rayons X utilisés en radiologie. Si le capteur est plus grand, il faut encore plus de temps.

Plutôt que de lire isolément un certain nombre de pixels de la matrice, dans l'invention, on propose avantageusement :
- de définir N régions d'intérêt, N au moins égal à deux, ayant un même nombre de pixels, qui sont toutes d'égales dimensions tant en nombre de lignes que de colonnes, formant ainsi des sous-matrices d'égales dimensions.
- de calculer la valeur moyenne du niveau de signal lu sur les pixels d'une ou plusieurs des régions d'intérêt ainsi définies ; et c'est la valeur moyenne qui est comparée à une valeur seuil prédéterminée, fonction des caractéristiques du capteur.

Comme les régions d'intérêt sont égales en nombre de pixels et également en nombre de lignes et de colonnes correspondantes de la matrice, on peut comparer les valeurs moyennes obtenues au même seuil ; et l'utilisation de plusieurs régions d'intérêt augmente la probabilité qu'au moins une de ces régions soit au moins en partie bien exposée au rayonnement et non pas cachée (derrière une dent ou autre masse absorbante). On obtient ainsi une bonne fiabilité de détection, tout en permettant un temps de boucle de test réduit, compatible de la détection que l'on souhaite faire. On peut préférer comparer à un seuil la valeur moyenne obtenue pour plusieurs régions d'intérêt, afin d'éviter d'arrêter trop tôt la source de rayons X. En effet, si une région d'intérêt correspond à une zone très peu absorbante, elle risque de provoquer trop tôt l'arrêt de la source de rayons X, conduisant à une sous-exposition d'autres régions plus intéressantes. En faisant la moyenne pour plusieurs régions d'intérêt on réduit ce risque.

A titre d'illustration, la figure 8 montre un capteur dans lequel on a défini sur la matrice de pixels, selon ces principes, 5 régions ROI₁ à ROI₅. Dans cet exemple, chaque région est une sous-matrice de 2³=8 pixels disposés en deux colonnes et quatre lignes.

L'intérêt de disposer de plusieurs régions est d'augmenter la chance de tester une région qui en partie au moins ne sera pas cachée du rayonnement. Les régions étant strictement égales en forme et surface, la valeur moyenne calculée sur ces régions est comparable.

Le nombre de pixels des régions d'intérêt et le nombre de régions d'intérêt que l'on peut définir doit tenir compte du temps nécessaire pour les lire : plus il y a de pixels à lire dans chaque région et plus il y a de régions, plus il faut de temps. Dans un exemple, si on définit 16 régions d'intérêt dont les dimensions sont de 16 lignes de largeur sur 16 lignes de hauteur, avec une lecture pixel à 5Mhz, il faudra typiquement compter 2.8 ms pour lire l'ensemble des pixels.

On peut avantageusement prévoir que la définition (nombre de pixels, de lignes et de colonnes), le nombre des régions d'intérêt et leurs positions respectives soit avantageusement paramétrables, en fonction notamment de l'objet (dent), du patient..., depuis l'ordinateur, via la liaison USB.

Une boucle de test de N régions d'intérêt qui peut être avantageusement implémentée dans un capteur selon ce principe est illustrée par l'organigramme de la figure 9 dans le cas où on veut déclencher une interruption de la source de rayons X dès le dépassement d'un seuil par une région d'intérêt ; si on veut détecter le dépassement pour la moyenne de plusieurs régions d'intérêt et non pas pour la région d'intérêt la plus exposée, on fera bien entendu un calcul de cette moyenne en vue d'élaborer le signal XAMC. Lorsque le début du rayonnement est détecté par le capteur, le signal de détection X-Start qui sert à activer la phase d'intégration, va également permettre d'activer la boucle de test. Cette activation peut être immédiate, ou après un certain temps prédéfini après le début de la phase d'intégration, pour que les pixels aient commencé à collecter un minimum de charges.

La boucle initialise alors un compteur du nombre de régions à tester et lance une boucle de lecture des pixels, calcul de la valeur moyenne sur les pixels et comparaison de la valeur moyenne à la valeur seuil prédéterminée, sur chacune des régions d'intérêt.

Dès que la valeur seuil est atteinte ou dépassée pour une région, la détection est faite : la boucle de test pour la phase d'intégration en cours se termine avec l'émission du signal de détection correspondant XAMC.

Tant que le résultat de la comparaison est négatif (seuil non atteint ou non dépassé), la boucle passe à la région d'intérêt suivante.

Quand la boucle a testé les N régions avec un résultat de comparaison négatif pour chacune des N régions, elle ré-initialise le compteur et le test reprend, jusqu'à détecter un dépassement par au moins une des régions, ou que la phase d'intégration se termine avec la période préprogrammée TINT. Dans ce cas où la boucle de test n'a pas permis de détecter la condition d'atteinte ou dépassement de la valeur seuil par au moins des régions d'intérêt, cela peut signifier que le temps d'exposition n'a pas été suffisant ; cela peut signifier aussi que l'un au moins des paramètres de définition des régions d'intérêt à savoir leur nombre, leurs dimensions ou leurs dispositions sur la matrice est à modifier. On prévoit avantageusement que ces paramètres sont programmables, via le dispositif (3) et la liaison USB, permettant de les adapter en fonction du patient et de l'objet à radiographier.

## Revendications

1. Procédé de prise d'image dans un système de radiologie médicale comprenant une source de rayonnement X externe (1) et un capteur d'image CMOS (2) relié par une liaison USB (4) en tant que périphérique USB d'un dispositif de traitement numérique (3) des données images du capteur, **caractérisé en ce que** le procédé comprend l'utilisation d'un équipement espion (5) connecté sur la liaison USB (4) entre le capteur (2) et le dispositif (3) pour une lecture et un décodage en continu des données transmises sur la liaison USB (4), d'une manière transparente pour le dispositif et pour le capteur, pour détecter dans le flux des données transmises, une donnée particulière (XAMC=1) représentant un signal de détection par le capteur qu'une dose de rayonnement suffisante a été reçue et, sur détection de cette donnée particulière, pour l'émission d'un signal électrique (Stop-X) vers la source de rayonnement pour stopper l'émission du rayonnement.

2. Procédé selon la revendication 1, **caractérisé en ce que** la détection de cette donnée particulière commande l'émission d'au moins un autre signal électrique (L) vers la source, pour commander un voyant d'affichage.

3. Procédé selon la revendication 1 ou 2, dans lequel ladite donnée particulière est une donnée transmise par le capteur sur la liaison USB selon un mode de transfert par interruption.

4. Procédé selon l'une des revendications précédentes, **caractérisé en ce qu'**il comprend :
- la définition de N régions d'intérêt (ROI₁,...ROI₅) sur la matrice de pixels du capteur, N entier au moins égal à deux, où les régions d'intérêt définies sont des sous-matrices de pixels d'égales dimensions, avec toutes le même nombre de lignes et le même nombre de colonnes,
- une lecture répétée non destructive des pixels de chacune des régions d'intérêt pendant la durée de la phase d'intégration du capteur, avec pour chacune des régions d'intérêt, le calcul d'une valeur moyenne du niveau de signal lu sur les pixels d'une ou plusieurs régions d'intérêt et la comparaison à une valeur seuil prédéterminée, et
- un signal de détection (XAMC) indiquant qu'une dose de rayonnement suffisante a été reçue est activé par le capteur et une information correspondante est transmise sur la liaison USB par le capteur dès que pour une ou plusieurs des N régions d'intérêt, la valeur moyenne calculée dépasse la valeur seuil.

5. Procédé selon la revendication 4, dans lequel la définition des N régions d'intérêt sur le capteur est programmable, via le dispositif (3) et la liaison USB.

6. Procédé selon l'une quelconque des revendications 1 à 5, pour la prise d'image radiologique dentaire au moyen du capteur d'image CMOS (2), qui est intra-oral et déjà placé à l'intérieur de la bouche d'un patient.

7. Système de radiologie médicale comprenant une source (1) de rayonnement X externe, un capteur (2) à matrice de pixels CMOS disposé en vis-à-vis de la source (1), et un dispositif (3) de traitement numérique des images fournies par le capteur, dans lequel le capteur est connecté comme périphérique USB dudit dispositif de traitement (3), par une liaison série USB (4), **caractérisé en ce que** :
- le capteur est configuré pour détecter qu'une dose suffisante de rayons X a été reçue et transmettre une donnée particulière correspondante sur la liaison USB ;
- un équipement espion (5) est connecté sur la liaison USB (4), entre le capteur (2) et le dispositif (3), ledit équipement ayant en outre une connexion électrique avec la source (1), pour transmettre au moins un signal électrique (Stop-X) à la source (1) pour commander l'arrêt du rayonnement X, ledit équipement (5) étant apte à détecter ladite donnée particulière dans le flux des données transmises sur la liaison USB, et activer le dit signal électrique (Stop-X) commandant l'arrêt de la source.

8. Système de radiologie médicale selon la revendication 7, dans lequel ladite connexion électrique de l'équipement espion (5) avec la source transmet au moins un autre signal (L) à la source (1) pour commander un voyant d'affichage.

9. Système de radiologie médicale selon la revendication 7 ou 8, **caractérisé en ce que** ledit équipement espion comprend au moins :
- un émetteur-récepteur USB (53) connecté aux fils de données (D-, D+) de la liaison USB, pour transmettre en sortie les données transitant sur la liaison USB sur un bus parallèle (DATA),
- un circuit logique programmable (54) programmé pour détecter ladite donnée particulière parmi lesdites données reçues du bus parallèle (DATA) et activer en sortie au moins un signal logique transmis à la source pour commander l'arrêt du rayonnement.

10. Système de radiologie médicale selon l'une quelconque des revendications 7 à 9, appliqué au domaine de la radiologie dentaire, dans lequel le capteur est un capteur d'image CMOS intra-oral.

## Patentansprüche

1. Verfahren zum Aufnehmen eines Bildes in einem medizinischen Radiologiesystem, das eine externe Röntgenquelle (1) und einen CMOS-Bildsensor (2) umfasst, der über eine USB-Verbindung (4) als USB-Peripheriegerät eines Geräts (3) zur digitalen Verarbeitung der Bilddaten des Sensors angeschlossen ist, **dadurch gekennzeichnet, dass** das Verfahren die Verwendung einer Spionausrüstung (5) beinhaltet, die über die USB-Verbindung (4) zwischen dem Sensor (2) und dem Gerät (3) angeschlossen ist zum kontinuierlichen Lesen und Decodieren der über die USB-Verbindung (4) übertragenen Daten in einer für das Gerät und den Sensor transparenten Weise, zum Detektieren, in dem übertragenen Datenstrom, eines bestimmten Datenelements (XAMC=1), das ein Signal zur Detektion durch den Sensor darstellt, dass eine ausreichende Strahlungsdosis empfangen wurde, und, bei Detektion dieses bestimmten Datenelements, zum Senden eines elektrischen Signals (Stop-X) an die Strahlungsquelle, um das Senden der Strahlung zu stoppen.

2. Verfahren nach Anspruch 1, **dadurch gekennzeichnet, dass** die Detektion dieses bestimmten Datenelements das Senden von mindestens einem weiteren elektrischen Signal (L) an die Quelle bewirkt, um eine Anzeigelampe zu steuern.

3. Verfahren nach Anspruch 1 oder 2, wobei das bestimmte Datenelement ein Datenelement ist, das vom Sensor über die USB-Verbindung in einem Unterbrechungsübertragungsmodus übertragen wird.

4. Verfahren nach einem der vorherigen Ansprüche, **dadurch gekennzeichnet, dass** es Folgendes beinhaltet:
- Definieren von N Bereichen von Interesse (ROI₁,...ROI₅) auf der Pixelmatrix des Sensors, wobei N eine ganze Zahl von mindestens zwei ist, wobei die definierten Bereiche von Interesse Submatrizen von Pixeln mit gleichen Abmessungen sind, die alle die gleiche Anzahl von Reihen und die gleiche Anzahl von Spalten aufweisen,
- wiederholtes zerstörungsfreies Lesen der Pixel jedes der Bereiche von Interesse während der Dauer der Integrationsphase des Sensors, dabei für jeden der Bereiche von Interesse Berechnen eines Mittelwertes des auf den Pixeln eines oder mehrerer Bereiche von Interesse gelesenen Signalpegels und Vergleichen desselben mit einem vorgegebenen Schwellenwert, und
- Aktivieren eines Detektionssignals (XAMC), das anzeigt, dass eine ausreichende Strahlungsdosis empfangen wurde, durch den Sensor und Übertragen einer entsprechenden Information über die USB-Verbindung durch den Sensor, sobald der berechnete Mittelwert für einen oder mehrere der N Bereiche von Interesse den Schwellenwert überschreitet.

5. Verfahren nach Anspruch 4, wobei das Definieren der N Bereiche von Interesse auf dem Sensor über das Gerät (3) und die USB-Verbindung programmierbar ist.

6. Verfahren nach einem der Ansprüche 1 bis 5 zum Aufnehmen von dentalen radiologischen Bildern mit Hilfe des CMOS-Bildsensors (2), der intraoral und bereits im Mund eines Patienten platziert ist.

7. Medizinisches Radiologiesystem, das eine externe Röntgenquelle (1), einen CMOS-Pixel-Array-Sensor (2), der gegenüber der Quelle (1) angeordnet ist, und ein Gerät (3) zum digitalen Verarbeiten der vom Sensor gelieferten Bilder, wobei der Sensor als USB-Peripheriegerät des Verarbeitungsgeräts (3) über eine serielle USB-Verbindung (4) angeschlossen ist, **dadurch gekennzeichnet, dass**:
- der Sensor zum Erkennen, dass eine ausreichende Röntgenstrahlendosis empfangen wurde, und zum Übertragen eines entsprechenden bestimmten Datenelements über die USB-Verbindung konfiguriert ist;
- eine Spionausrüstung (5) über die USB-Verbindung (4) zwischen dem Sensor (2) und dem Gerät (3) angeschlossen ist, wobei die Ausrüstung außerdem eine elektrische Verbindung mit der Quelle (1) aufweist, um mindestens ein elektrisches Signal (Stop-X) an die Quelle (1) zu übertragen, um das Stoppen der Röntgenstrahlung zu steuern, wobei die Ausrüstung (5) das bestimmte Datenelement in dem über die USB-Verbindung übertragenen Datenstrom detektieren und das das Stoppen der Quelle (1) steuernde elektrische Signal (Stop-X) aktivieren kann.

8. Medizinisches Radiologiesystem nach Anspruch 7, wobei die elektrische Verbindung der Spionausrüstung (5) mit der Quelle mindestens ein weiteres Signal (L) an die Quelle (1) überträgt, um eine Anzeigelampe zu steuern.

9. Medizinisches Radiologiesystem nach Anspruch 7 oder 8, **dadurch gekennzeichnet, dass** die Spionausrüstung mindestens Folgendes umfasst:
- einen USB-Transceiver (53), der an die Datenleitungen-(D-, D+) der USB-Verbindung angeschlossen ist, zum Übertragen, am Ausgang, der Daten über die USB-Verbindung auf einem parallelen Bus (DATA),
- eine programmierbare Logikschaltung (54), programmiert zum Detektieren des bestimmten Datenelements unter den vom parallelen Buss (DATA) empfangenen Daten und zum Aktivieren, am Ausgang, mindestens eines an die Quelle übertragenen Logiksignals zum Steuern des Stoppens der Strahlung.

10. Medizinisches Radiologiesystem nach einem der Ansprüche 7 bis 9, angewendet auf den Bereich der dentalen Radiologie, wobei der Sensor ein intraoraler CMOS-Bildsensor ist.

## Claims

1. A method for taking images in a medical radiology system comprising an external x-ray source (1) and a CMOS image sensor (2) that is linked via a USB link (4) as USB peripheral of a device (3) for digitally processing the image data of the sensor, **characterized in that** the method comprises the use of a tracking device (5), that is connected over the USB link (4) between the sensor (2) and the device (3), for continuously reading and decoding the data transmitted over the USB link (4), in a way that is transparent to the device and to the sensor, to detect, in the transmitted data stream, one particular datum (XAMC=1) representing a signal indicating detection, by the sensor, that a sufficient dose of radiation has been received and, on detection of this particular datum, to send an electrical signal (Stop-X) to the radiation source to stop the emission of the radiation.

2. The method according to claim 1, **characterized in that**, on detection of this particular datum, at least one other electrical signal (L) is sent to the source to command a display indicator light.

3. The method according to claim 1 or claim 2, wherein said particular datum is a datum transmitted by the sensor over the USB link in an interrupt transfer mode.

4. The method according to one of the preceding claims, **characterized in that** it comprises:
- the definition of N regions of interest (ROI₁,...ROI₅) in the matrix of pixels of the sensor, N being an integer at least equal to two, where the defined regions of interest are sub-matrices of pixels of equal size, all with the same number of rows and the same number of columns,
- repeated and nondestructive read-out of the pixels of each of the regions of interest during the duration of the integration phase of the sensor, with, for each of the regions of interest, calculation of an average value of the signal level read from the pixels of one or more regions of interest and comparison to a preset threshold value, and
- a detection signal (XAMC), indicating that a sufficient dose of radiation has been received, is activated by the sensor and a corresponding piece of information is transmitted over the USB link by the sensor as soon as, for one or more of the N regions of interest, the calculated average value exceeds the threshold value.

5. The method according to claim 4, wherein the definition of the N regions of interest in the sensor is programmable, via the device (3) and the USB link.

6. The method according to any one of claims 1 to 5 for taking dental radiological images by means of the CMOS image sensor, which is intra-oral and is already placed in the mouth of a patient.

7. A medical radiology system comprising an external x-ray source (1), a CMOS sensor (2) with a matrix of pixels, which sensor is placed facing the source (1), and a device (3) for digitally processing the images delivered by the sensor, wherein the sensor is connected as a USB peripheral of said processing device (3), via a serial USB link (4), **characterized in that**:
- the sensor is configured to detect that a sufficient dose of x-rays has been received and to transmit a corresponding particular datum over the USB link;
- a tracking device (5) is connected to the USB link (4) between the sensor (2) and the device (3), said tracking device furthermore having an electrical connection to the source (1), in order to transmit at least one electrical signal (Stop-X) to the source (1) to command the stoppage of the x-ray source, said tracking device (5) being able to detect said particular datum in the data stream transmitted over the USB link, and to activate said electrical signal (Stop-X) commanding the stoppage of the source.

8. The medical radiology system according to claim 7, wherein said electrical connection of the tracking device (5) with the source transmits at least one other signal (L) to the source (1) to command a display indicator light.

9. The medical radiology system according to claim 7 or 8, **characterized in that** said tracking device comprises at least:
- one USB transceiver (53) that is connected to the data wires (D-, D+) of the USB link to transmit as output the data transiting over the USB link on a parallel bus (DATA),
- a programmable logic circuit (54) that is programmed to detect said particular datum among said data received from the parallel bus (DATA) and to activate as output at least one logic signal that is transmitted to the source to command the stoppage of the radiation.

10. The medical radiology system according to any one of claims 7 to 9 applied in the field of dental radiology, wherein the sensor is an intra-oral CMOS image sensor.
